# EUROPEAN PATENT SPECIFICATION

(11) **EP 1 776 452 B1**
(45) Date of publication and mention of the grant of the patent: **12.11.2008**
(21) Application number: 05776723.8
(22) Date of filing: 19.08.2005
(51) Int. Cl.: C12N 5/00, A61K 9/50, B01L 3/00

(54) **WASHING APPARATUS**
WASCHVORRICHTUNG
DISPOSITIF DE LAVAGE

(30) Priority: 20.08.2004 EP 04019786
(43) Date of publication of application: 25.04.2007
(73) Proprietor: Bayer Schering Pharma Aktiengesellschaft, 13353 Berlin (DE)
(72) Inventor: VORHOLZ, Jessica, F., M., 10625 Berlin (DE); RYBKA, Maja, I., J., 10409 Berlin (DE)
(74) Representative: Gross, Felix
(86) International application number: PCT/EP2005/008993
(87) International publication number: WO 2006/018312

(56) References cited:
- EP-A- 0 608 153
- US-A- 4 435 505
- US-A- 6 103 271

## Description

The present invention relates to a washing apparatus, in particular to a washing apparatus for washing cell carrier microcapsules carrying cells.

Cell carrying microcapsules are among others described in EP 0 554 352 A or in EP O 222 718 A which are incorporated hereinto by reference. The microcapsules may be made of glass, other silicon oxides, polystyrene, polypropylene, polyethylene, polyacrylamide, polycarbonate, polypentene, acrylonitride polymers, nylon, amylases, collagen, polysaccharides, and magnetite beads or formed of a gel or gelatin. The typical size is 10 to 500 pm. These microcapsules are used to implant cells onto the microcapsules or into the microcapsules. Such microcapsules can be used to be implanted into sensitive tissue, e.g. the brain in order to provide a medical treatment.

From US 4,435,505 an apparatus for blood sample treatments is known. The apparatus comprises an upper chamber having a bottom wall formed with a central depending spout. The top wall of the upper chamber is formed with an upstanding inlet conduit connected to a flexible plastic conduit leading to a flexible bag containing lysing solution. A further upstanding inlet conduit is connected to a further flexible plastic conduit leading to a further flexible bag containing a culture medium. Below the upper chamber a cylindrical bottom chamber is provided for receiving the content of the upper chamber. Vacuum can be applied to the bottom chamber in order to promote the transfer of the content of the upper chamber to the lower chamber.

EP 608 153 A1 describes a reaction apparatus with automating dosing system, especially for DNA extraction. The apparatus comprises a storing container, a washing container with a plunger and a space with a plunger suitable for storing a washing solution.

From EP 0 608 153 A1 an apparatus can be taken for automatically applying a reaction agent, in particular for extraction for DNA plasma. A reaction container is applied with the reaction agent from a further container and with a culture from another container. In order to avoid that the culture adheres to a filter medium, a turbulent flow is desired. A piston is provided for pressing the reaction agent directly through the filter medium.

From US 6,103,271 an apparatus and a method can be taken for forming spherical multi lamellar microcapsules having alternating hydrophilic and hydrophobic liquid layers, surrounded by flexible, semi-permeable hydrophobic or hydrophilic outer membranes which can be tailored to control the diffusion rate. Low shear mixing and liquid-liquid diffusion processes are used. The microcapsules are formed, washed and filtered.

A typical example are gelatin spheres onto which neurons or neuronal-like cells or retinal pigment epithelial cells are deposited. If retinal pigment ephitelial cells are attached or deposited on cell carrier microcapsules of spherical crosslinked gelantine micro-carriers, the term "Spherarnine®'l is used.

Spheramine® is a very delicate product. The cell carrier microcapsules with the cells thereon are mechanically very unstable. If high shear forces are applied to the Spheramine®, it is likely that the cells detach from the cell carrier microcapsules, so that the product becomes worthless, the Spheramine® loses its function, after further implantation. Therefore, any treatment which applies shear forces has to be avoided.

Cell carrier microcapsules carrying cells and in particular Spheramine® are either stored at -196°C. In order to survive this cooling treatment, the cell carrier microcapsules carrying cells have to be kept in a particular freezing buffer solution. However, this freezing buffer solution is highly toxical. Therefore, if the cell carrier microcapsules carrying cells, which were stored at -196°C are warmed up again to be prepared for use and application to a patient, the freezing buffer solution has to be removed quickly by washing the cell carrier microcapsules.

Alternatively, the cell carrier microcapsules carrying cells can be kept in a hibernation buffer solution at 2 to 8°C. However, this hibernation buffer solution is also toxical. Therefore, before the application of the cell carrier microcapsules carrying cell to a patient, they have to be washed again.

In the following the term "storage buffer solution" will be used for both alternatives.

Up to now the cell carrier microcapsules carrying cells kept in their storage buffer solution are set into a tube, a washing solution is added by a pipette, the tube is agitated or vibrated with care. Sedimentation is awaited. The liquid is removed from the tube until the meniscus just touches the top of the remaining slurry. New washing solution is added and the above procedure is repeated.

This is a slow and cumbersome method to wash the cell carrier microcapsules carrying cells. The removal of the washing liquid is difficult. Attempts were made to use centrifuges in order to speed up the proceeding. However, it was found that the shear forces acting on the cell carrier microcapsules carrying cells, were too strong. The cells came off from the cell carrier microcapsules.

More important, tubes and pipettes are not "medical products". They are not admitted for direct medical application. Only syringes are admitted as medical products. But the use of syringes is even more cumbersome. In addition, when the cell carrier microcapsules carrying cells are kept in tubes and are treated with pipettes or syringes, they are exposed to the atmosphere. This leads to the great danger of contamination, which is not acceptable.

Therefore, it is an object of the present invention to provide a washing apparatus for washing cell carrier microcapsules carrying cells, in which the washing can take place more efficient and smooth so that the cell carrier microcapsules carrying cells can be washed quickly, sterile and without damage to them.

This object is solved by a washing apparatus for washing cell carrier microcapsules carrying cells as is claimed in claim 1.

Preferred developments of the washing apparatus are defined in the dependent claims.

The washing apparatus of the present invention has the advantage that the washing takes place continuously so that it is much more effective than the above discussed method. The washing apparatus has the great advantage that no centrifugalising is necessary so that the cell carrier microcapsules are not damaged. The washing apparatus has the advantage that it can be sterilized. The time of the exposure to atmosphere can be minimized. Thus, the loss of sterility in a medical application is minimized. The washing apparatus of the present invention has the advantage that the difficult step of removing the liquid from the tube can be facilitated, the risk of a product loss is decreased.

Aspects of the invention will be described in more detail with reference to the accompanying drawings, in which:
- Fig. 1: shows a first embodiment of the washing apparatus of an aspect of the present invention;
- Fig. 2: shows a second embodiment of the washing apparatus of an aspect of the present invention;
- Fig. 3: shows the washing apparatus of the second embodiment wherein a plunger is added to the washing container;
- Figs. 4a: to 4d show different plunger shapes for the plunger in the washing container;
- Figs. 5a: to 5e show different steps of washing cell carrier microcapsules carrying cells with the washing apparatus according to the second embodiment; and
- Figs. 6a: to 6d show different steps of washing cell carrier microcapsulses carrying cells with a washing apparatus according to a third embodiment.

### First embodiment of an aspect of the invention

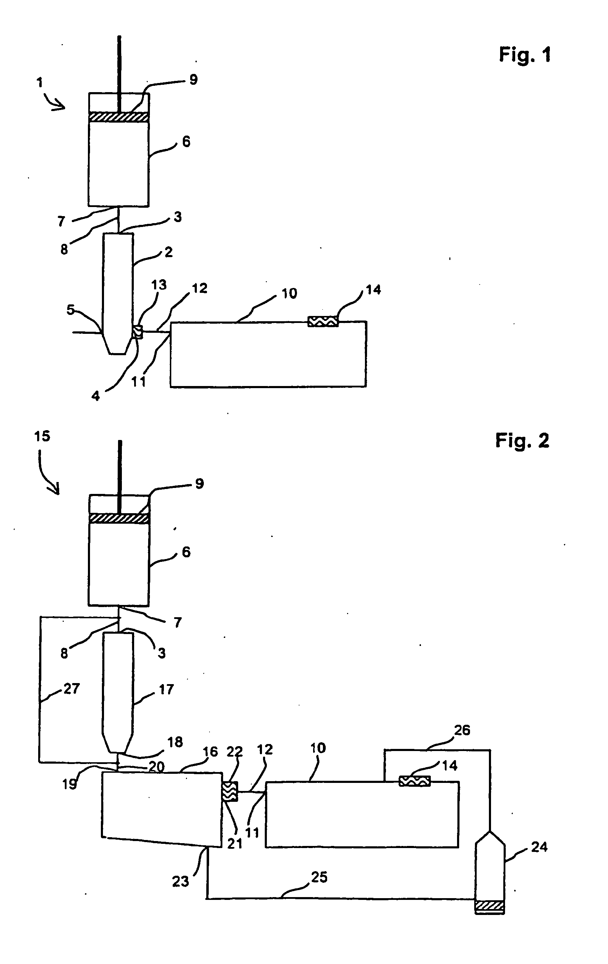

In the following a first embodiment of a washing apparatus 1 of the present invention will be described under reference to Fig. 1. The washing apparatus 1 comprises a product container 2 as a central unit. The product container 2 is used to store cell carrier microcapsules carrying cells like Spheramine® during long terms in the range up to several months. The product container 2 is adapted to take up the cell carrier micro cells in a freezing buffer solution at a temperature of -196°C or a hibernation buffer solution (storage buffer solution). In order to survive such deep temperatures, the product container 2 is formed of polyolefin by molding or of glass. It is also possible to use a syringe, in particular a silicone free syringe.

The product container 2 comprises in a position for use at an upper end an inlet 3 and at a lower section a first outlet 4. Also at the lower section of the product container 2 a second outlet 5 is provided, which will be described in more detail later on.

The washing apparatus 1 includes a storage container 6 for containing a washing solution for the cell carrier microcapsules. The storage container is made of polyolefin or glass. The washing solution is adapted to wash the cell carrier microcapsules carrying cells, wherein the washing solution does not exhibit any dangerous property for the intended use of the cell carrier microcapsules carrying cells. The washing solution is typically Hanks Balanced Salt Solution (HBSS). The storage container comprises an outlet 7. The outlet 7 of the storage container is connected with the inlet 3 of the product container by a tube 8.

Inside the storage container 6 a plunger 9 is provided for expelling the washing solution out of the storage container 6 through the outlet 7 and into the inlet 3 of the product container 2 into the product container.

The washing apparatus 1 comprises a waste container 10. The waste container 10 comprises an inlet 11. The inlet 11 of the waste container 10 is connected to the first outlet 4 of the product container by a tube 12. At the end of the tube 12 facing the outlet 4 of the product container 2 a filter 13 is provided. The filter 13 has a pore size of less than 50 µm, preferably 20 to 40 µm, more preferably 30 µm. The pore size is determined in dependency of the size of the microcapsules.

In a position intended for use the waste container 10 comprises at the top an opening into which an optional filter 14 is inserted. The filter 14 has a pore size of 0,1 µm to 1,0 µm, preferably 0,15 to 0,3 µm, more preferably 0,2 µm. The filter 14 serves for venting the waste container 10.

The storage container 6 is detachable from the product container 2. The waste container 10 is detachable from the product container 2.

For connection between the tubes and the containers typically Luer connections are used.

According to a modification, the product container 2 and the waste container 10 are formed as a unit. In such a case the tube 12 is not necessary. The filter 13 is provided at the connection between the product container 2 and the waste container 10.

In the following the method of operating the washing apparatus 1 of Fig. 1 will be described briefly. The product container 2 is used for storing the cell carrier microcapsules carrying cells during long term storage or during transport. In such cases the cell carrier microcapsules carrying cells are kept in a freezing buffer solution or hibernation buffer solution, respectively. Both types of buffer solution will be called storage buffer solution. For the freezing buffer solution a solution of Dimethylsulfoxid (DMSO) is preferred. For the hibernation buffer solution typically a solution containing Fetal Calf Serum (FCS), Phosphate Buffered Saline (PBS) or Dulbecco's Modified Eagle Media is used. Both types of solution have to be removed from the cell carrier microcapsules carrying cells before use. Therefore, the product container 2 is connected to the storage container 6 and to the waste container 10. The storage container 6 stores the washing solution which is not an obstacle to the use of the cell carrier microcapsules carrying cells. In the intended position of use the plunger 9 is moved downwards to expel the washing solution from the storage container 6 into the product container 2. The washing solution flows through the product container 2 in a substantially laminary flow and cleans the cell carrier microcapsules carrying cells without applying shear forces to the cell carrier microcapsules carrying cells. The washing solution takes with it the storage buffer solution. The washing solution flows through the filter 13 into the waste container 10 expelling the atmosphere in the waste container 10 through the filter 14. The filter 13 comprises pores small enough to prevent the cell carrier microcapsules carrying cells to pass through. Therefore, the cell carrier microcapsules carrying cells are maintained in the product container 2 in a cleaned condition. The flow of the washing solution can be adjusted such that no damage is done to the cell carrier microcapsules carrying cells.

After washing the cell carrier microcapsules carrying cells, the cleaned cell carrier microcapsules carrying cells can be extracted through the second outlet 5 of the product container 2.

### Second embodiment of an aspect of the invention

In the following a second embodiment of a washing apparatus 15 will be described referring to Fig. 2.

The second embodiment of the washing apparatus 15 differs from the first embodiment of the washing apparatus 1 in that additional a washing container 16 is provided. In the following description those parts of the washing apparatus 15 which are the same as of the washing apparatus 1 of the first embodiment will not be repeated.

The washing apparatus 15 comprises a product container 17 which has a single outlet 18. The single outlet 18 is connected with an inlet 19 of the washing container 16 through a tube 20. On the other side, the washing container 16 comprises a first outlet 21 corresponding to the first outlet 4 of the product container 2 of the first embodiment. At the first outlet 21 of the washing container 16 of the second embodiment a filter 22 is provided which corresponds in its properties to the filter 13 of the first embodiment. The first outlet 21 of the washing container 16 is connected with the waste container 10 through a pipe 12 as in the first embodiment.

The washing container 16 comprises a second outlet 23. The second outlet 23 of the washing container 16 is connectable through a tube, a hose or similar 25 with a cartridge 24. The cartridge 24 serves for receiving the cell carrier microcapsules carrying cells being cleaned or washed. The cartridge 24 will be used when the cell carriers microcapsules carrying cells will be applied.

In Fig. 2 the tube 25 is shown to have a certain extent. However, it is also possible to attach the cartridge 24 directly at the washing container 16.

### First modification

According to a first modification of the second embodiment the cartridge 24 is in the intended position of use at the upper side connected with an overflowed tube 26 which again is connected to the waste container 10. The overflow tube 26 is intended to guide excessive product from the cartridge 24 to the waste container 10.

### Second modification

According to a second modification of the second embodiment an additional bypass 27 is connected between the tube 8 connecting the storage container 6 and the product container 17 and the tube 20 connecting the product container 17 and the washing container 16. The additional bypass tube 27 serves for guiding washing solution from the storage container 6 directly to the washing container 16 if it appears to be necessary to intensify the washing in the washing container 16.

### Third modification

According to a third modification of the second embodiment in the washing container 16 an additional plunger 28 is provided. The additional plunger 28 in the washing container 16 facilitates the transport of the cell carrier microcapsules carrying cells being cleaned from the washing container 16 to the cartridge 24.

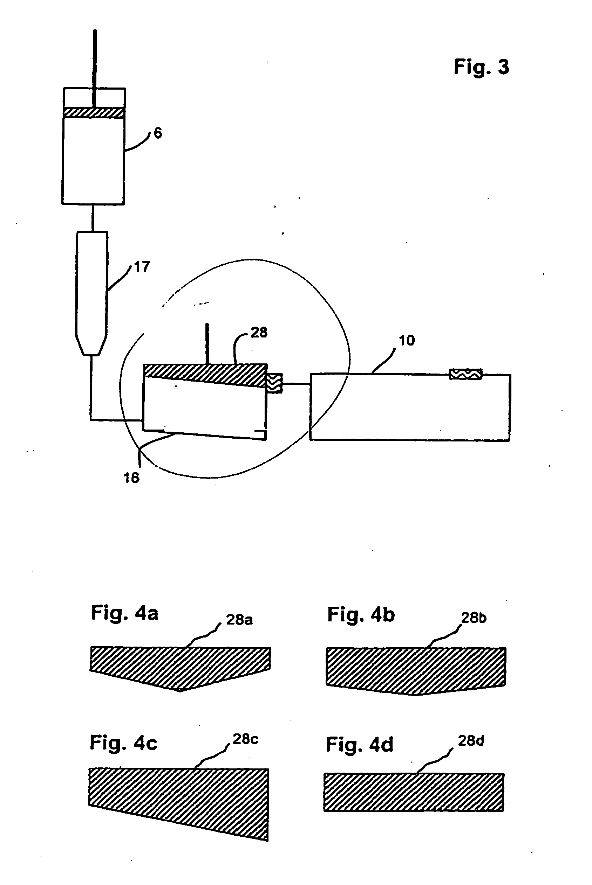

As shown in Figs. 4a to 4d different shapes of the additional plunger 28a, 28b, 28c and 28d may be used. The plunger 28c corresponds to the plunger 28 shown in Fig. 3. However, depending on the shape of the bottom of the washing container 16 in the position of the intended use, the additional plunger can have a shape as is shown in Figs. 4a, 4b, and 4d which is adapted to a different bottom form as is shown in Fig. 3. This makes it possible to empty washing container 16 very effectively.

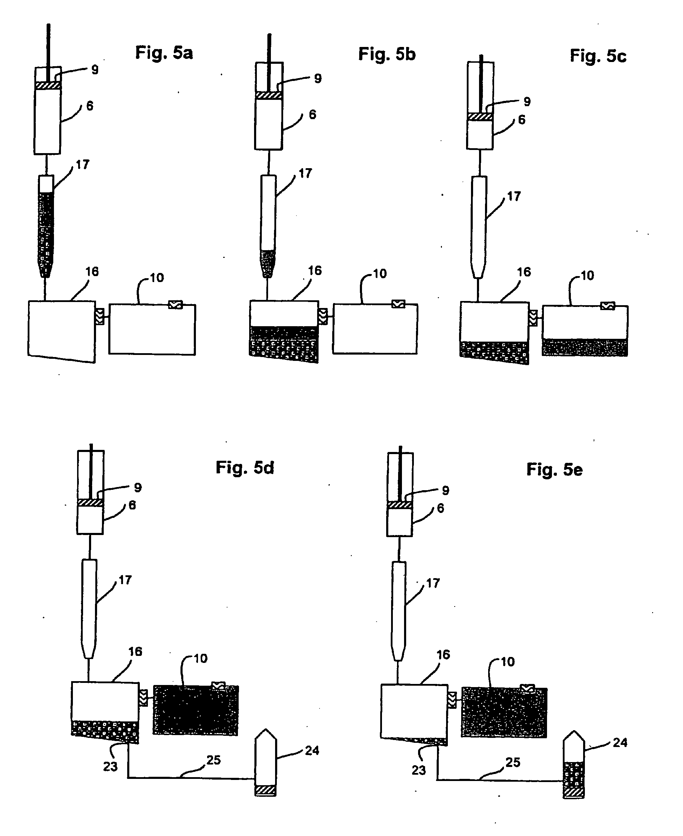

Now, an operating method of the washing apparatus of the second embodiment will be described referring to Fig. 5a to 5e. As is shown in Fig. 5a the storage container 6 is filled with the washing solution. The product container 17 is filled with the cell carrier microcapsules carrying cells at the bottom thereof, the cell carrier microcapsules carrying cells are distributed in the storage buffer solution. If the cell carrier microcapsules carrying cells are kept in the freezing buffer solution at -196°C the freezing buffer solution has to be thawed before.

As is shown in Fig. 5b, the piston 9 of the storage container 6 is moved downward to expel the washing solution from the storage container 6. At the same time the cell carrier microcapsules carrying cells are expelled from the product container 17 into the washing container 16. As can be seen from Fig. 5c also the storage buffer solution is removed from the product container 17 firstly into the washing container 16 and thereafter into the waste container 10. The washing solution in the storage container 6 is used to wash the cell carrier microcapsules carrying cells in the washing container 16. The washing solution is transferred together with the storage buffer solution to the waste container 10.

As can be seen in Fig. 5d the cartridge 24 for receiving the cell carrier microcapsules carrying cells is connected to the second outlet 23 of the washing container 16.

Finally, as can be seen in Fig. 5e the cell carrier microcapsules carrying cells are expelled from the washing container 16 through the tube or hose 25. Thereafter, the cartridge 24 is ready for use for applying the cell carrier microcapsules carrying cells for the intended purpose. Expelling the cell carrier microcapsules carrying cells from the washing container 16, can be assisted by the additional plunger 28, which is not shown for sake of simplification in Figs. 5a to 5e.

Though it is not shown in Fig. 2, the tube 8 connecting the storage container 6 with the product container 17 may include a venting device between the product container 17 and the storage container 6 so that the atmosphere contained in the product container 17 can escape.

### Third embodiment of an aspect of the invention

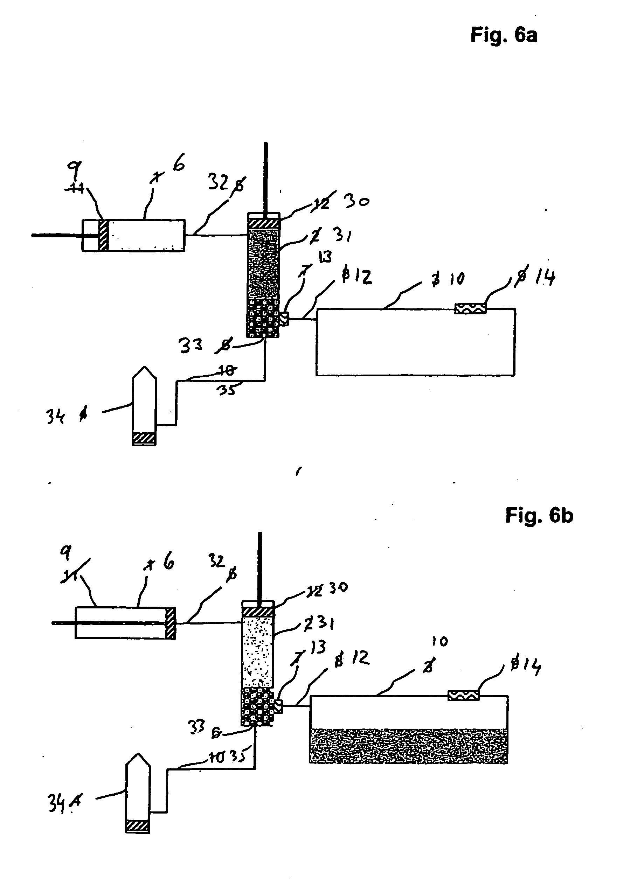

In the following a third embodiment of a washing apparatus 29 will be described referring to Figs. 6a to 6d.

The third embodiment of the washing apparatus 29 differs from the first embodiment of the washing apparatus 1 in that a plunger 30 is provided in a product container 31. In the following description those parts of the washing apparatus 29 which are the same as of the washing apparatus 1 of the first embodiment will not be repeated.

The storage container 6 is connected with the product container 31 through a tube 32. The tube 32 is connected with the product container 31 downstream of the plunger 30. The product container 31 comprises an outlet 33 at the bottom side thereof. The outlet 33 is connected with a cartridge 34 by a tube 35. The other features are the same as in the first embodiment.

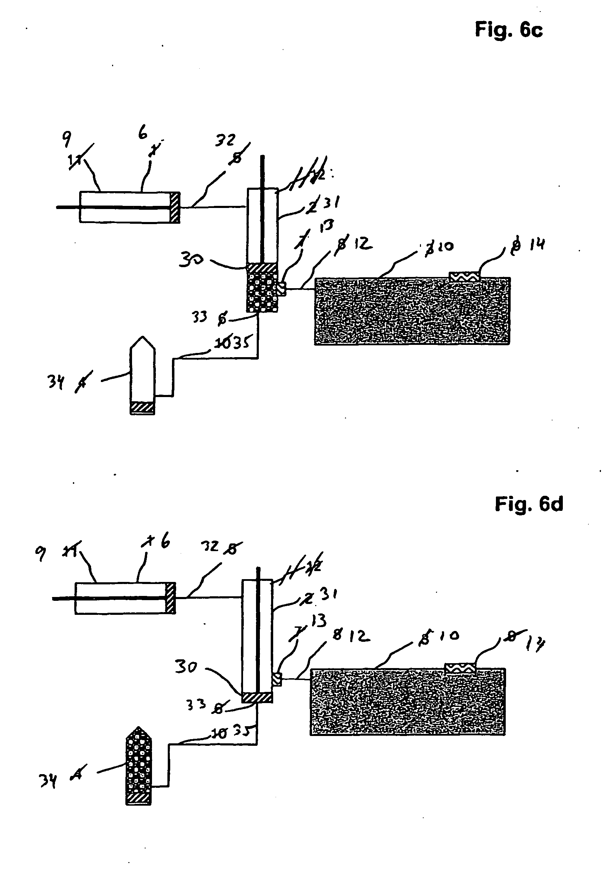

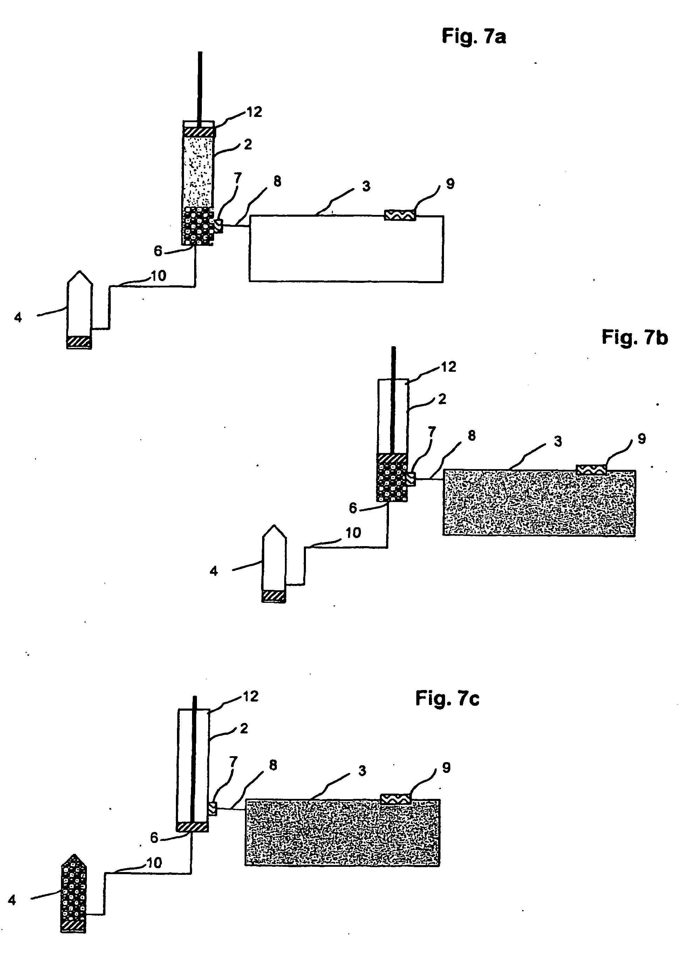

In the following the operation of the washing apparatus 29 of the third embodiment will be described using the sequence of the Figures 6a, 6b, 6c, and 6d.

Initially, as shown in Fig. 6a, the storage container 6 is filled with the washing solution for the cell carrier microcapsules. The product container 31 is filled with cell carrier microcapsules carrying cells like Spheramine^{®} in a freezing buffer solution or a hibernation buffer solution (storage buffer solution).

Thereafter, as shown in Fig. 6b the plunger 9 is used to expel the washing solution from the storage container 6 into the product container 31 and through the product container 31 through the filter 13 into the waste container 10. Thereby, the cell carrier microcapsules carrying cells are washed by the washing solution of the storage container 6. At the end of the washing process the product container 31 is filled with the washed cell carrier microcapsules carrying cells and remaining washing solution.

Thereafter, the plunger 30 in the product container 31 is moved to expel the remaining washing solution in the product container 31 into the waste container 10. At the end of this removal step, as is shown in Fig. 6c, all the washing solution has been removed from the product container 31, only the cell carrier microcapsules carrying cells remain in the product container 31.

Thereafter, in order to assist the removal of the cell carrier microcapsules carrying cells from the product container 31 into the cartridge 34, the plunger 30 is further moved to the end of the product container 31 so that all cell carrier microcapsules carrying cells are transferred into the cartridge 34, as is shown in Fig. 6d.

In Figs. 6a to 6d a tube 35 is shown connecting the outlet of the product container 31 with the cartridge 34. However, the cartridge 34 can be provided close to the product container 31, in particular, the cartridge 34 can be provided directly at the product container 31, so that no tube 35 is necessary.

### Washing Apparatus

| Char | Feature |
|---|---|
| 1 | Washing apparatus 1. AF |
| 2 | Product container |
| 3 | Inlet of product container 2 |
| 4 | First outlet of product container 2 |
| 5 | Second outlet of product container 2 |
| 6 | Storage container |
| 7 | Outlet of storage container 6 |
| 8 | Tube between product container 2 and storage container 6 |
| 9 | Plunger in storage container 6 |
| 10 | Waste container |
| 11 | Inlet of waste container 10 |
| 12 | Tube between product container 2 and waste container 10 |
| 13 | Filter at outlet of product container 2 |
| 14 | Filter at outlet of waste container 10 |
| 15 | Washing apparatus 2. AF |
| 16 | Washing container |
| 17 | Product container |
| 18 | Outlet of product container 17 |
| 19 | Inlet of washing container 16 |
| 20 | Tube between product container 17 and washing container 16 |
| 21 | First outlet of washing container 16 |
| 22 | Filter at outlet of washing container 16 |
| 23 | Second outlet of washing container 16 |
| 24 | Cartridge |
| 25 | Tube, hose between washing container 16 and cartridge 24 |
| 26 | Overflow tube of cartridge 24 |
| 27 | Additional by-pass tube for product container 17 |
| 28 | Additional plunger in washing container 16 |
| 29 | Washing apparatus 3. AF |
| 30 | Plunger in product container 31 |
| 31 | Product container |
| 32 | Tube between storage container 6 and product container 31 |
| 33 | Outlet of product container 31 |
| 34 | Cartridge |
| 35 | Tube between product container 31 and cartridge 34 |

## Claims

1. A washing apparatus (1) for washing cell carrier microcapsules carrying cells comprising:
a product container (17) for storing the cell carrier microcapsules in a storage solution, the product container (17) having an inlet (3) and an outlet (18) ;
a storage container (6) for storing a washing solution, the storage container (6) having an outlet (7) connected with the inlet (3) of the product container (17); and
a washing container (16) for receiving the cell carrier microcapsules in the storage solution and for washing the cell carrier microcapsules;
wherein the washing container (16) has an inlet (19), the washing container (16) being connected with the inlet (19) thereof to the outlet (18) of the product container (17,
wherein a first plunger (9) is provided in the storage container (6) for expelling the washing solution from the storage container (6) into the product container (17),
wherein a second plunger (30) is provided in the product container (17) for expelling the storage solution and the cell carrier microcapsules out of the product container (17),
wherein the washing container (16) comprises a third plunger (28) at the top section thereof for expelling the cell carrier microcapsules, the plunger (28) having a bottom shape complementary to a bottom shape of the washing container (16).

2. The washing apparatus according to claim 1,
wherein the washing container (17) has a first outlet (21),
the washing container (17) being connected with the first outlet (21) thereof to an inlet (11) of a waste container (10).

3. The washing apparatus according to claim 2,
wherein the waste container (10) is connected to the washing container (16) through a filter (22), the filter (22) preferable having a pore size of less than 50 µm, more preferably of about 20 to 40 µm, most preferably about 30 µm.

4. The washing apparatus according to one of claims 1 to 3,
wherein the washing container (16) has a second outlet (23) for connecting to a cartridge (24) for receiving the cell carrier microcapsules.

5. The washing apparatus according to claim 4,
wherein the third plunger (28) facilitates the transport of the cell carrier microcapsules from the washing container (16) to the cartridge (24).

6. The washing apparatus according to one of claims 1 to 5,
wherein a bypass tube (27) is provided connecting the storage container (6) to the washing container (16).

7. The washing apparatus according to one of claims 2 to 6,
wherein an overflow tube (26) is provided connecting a cartridge (24) to the waste container (10).

8. The washing apparatus according to one of claims 2 to 7,
wherein the waste container (10) comprises an opening with a filter (14) for venting, the filter (14) preferably having a pore size of 0.1 to 1.0 µm, more preferable of about 0.15 to 0.3 µm, most preferably, of about 0.2 µm.

## Patentansprüche

1. Waschvorrichtung (1) zum Waschen von Zellträger-Mikrokapseln, welche Zellen tragen, umfassend:
einen Produktbehälter (17) zum Bevorraten der Zellträger-Mikrokapseln in einer Vorratslösung, wobei der Produktbehälter (17) eine Einlassöffnung (3) und eine Auslassöffnung (18) aufweist;
einen Vorratsbehälter (6) zum Bevorraten einer Waschlösung, wobei der Vorratsbehälter (6) eine Auslassöffnung (7) aufweist, die mit der Einlassöffnung (3) des Produktbehälters (17) verbunden ist; und
einen Waschbehälter (16) zum Aufnehmen der Zellträger-Mikrokapseln in der Vorratslösung und zum Waschen der Zellträger-Mikrokapseln;
wobei der Waschbehälter (16) eine Einlassöffnung (19) aufweist, wobei der Waschbehälter (16) über seine Einlassöffnung (19) mit der Auslassöffnung (18) des Produktbehälters (17) verbunden ist,
wobei in dem Vorratsbehälter (6) ein erster Kolben (9) zum Ausstoßen der Waschlösung aus dem Vorratsbehälter (6) in den Produktbehälter (17) vorgesehen ist,
wobei in dem Produktbehälter (17) ein zweiter Kolben (30) zum Ausstoßen der Vorratslösung und der Zellträger-Mikrokapseln aus dem Produktbehälter (17) vorgesehen ist,
wobei der Waschbehälter (16) an seinem oberen Abschnitt einen dritten Kolben (28) zum Ausstoßen der Zellträger-Mikrokapseln umfasst, wobei der Kolben (28) eine Bodenform aufweist, die zu der Bodenform des Waschbehälters (16) komplementär ist.

2. Waschvorrichtung gemäß Anspruch 1,
wobei der Waschbehälter (16) eine erste Auslassöffnung (21) aufweist,
wobei der Waschbehälter (16) über seine erste Auslassöffnung (21) mit einer Einlassöffnung (11) eines Abfallbehälters (10) verbunden ist.

3. Waschvorrichtung gemäß Anspruch 2,
wobei der Abfallbehälter (10) über ein Filter (22) mit dem Waschbehälter (16) verbunden ist, wobei das Filter (22) vorzugsweise eine Porengröße von weniger als 50 µm, weiter bevorzugt etwa 20 bis 40 µm, äußerst bevorzugt etwa 30 µm, aufweist.

4. Waschvorrichtung gemäß einem der Ansprüche 1 bis 3, wobei der Waschbehälter (16) eine zweite Auslassöffnung (23) zum Verbinden mit einer Kartusche (24) zum Aufnehmen der Zellträger-Mikrokapseln aufweist.

5. Waschvorrichtung gemäß Anspruch 4,
wobei der dritte Kolben (28) die Überführung der Zellträger-Mikrokapseln von dem Waschbehälter (16) zu der Kartusche (24) unterstützt.

6. Waschvorrichtung gemäß einem der Ansprüche 1 bis 5,
wobei eine Nebenleitung (27), welche den Vorratsbehälter (6) mit dem Waschbehälter (16) verbindet, vorgesehen ist.

7. Waschvorrichtung gemäß einem der Ansprüche 2 bis 6,
wobei eine Überlaufleitung (26), welche eine Kartusche (24) mit dem Abfallbehälter (10) verbindet, vorgesehen ist.

8. Waschvorrichtung gemäß einem der Ansprüche 2 bis 7,
wobei der Abfallbehälter (10) eine Öffnung mit einem Filter (14) zum Belüften umfasst, wobei das Filter (14) vorzugsweise eine Porengröße von 0,1 bis 1,0 µm, weiter bevorzugt etwa 0,15 bis 0,3 µm, äußerst bevorzugt etwa 0,2 µm, aufweist.

## Revendications

1. Appareil de lavage (1) destiné à laver des microcapsules de support de cellule portant des cellules comprenant :
un réservoir de produit (17) pour stocker les microcapsules de support de cellule dans une solution de stockage, le réservoir de produit (17) présentant une entrée (3) et une sortie (18) ;
un réservoir de stockage (6) pour stocker une solution de lavage, le réservoir de stockage (6) ayant une sortie (7) raccordée à l'entrée (3) du réservoir de produit (17) ; et
un réservoir de lavage (16) pour recevoir les microcapsules de support de cellule dans la solution de stockage et pour laver les microcapsules de support de cellule ;
dans lequel le réservoir de lavage (16) présente une entrée (19), le réservoir de lavage (16) étant raccordé à son entrée (19) vers la sortie (18) du réservoir de produit (17),
dans lequel un premier plongeur (9) est prévu dans le réservoir de stockage (6) pour expulser la solution de lavage depuis le réservoir de stockage (6) jusque dans le réservoir de produit (17),
dans lequel un second plongeur (30) est prévu dans le réservoir de produit (17) pour expulser la solution de stockage et les microcapsules de support de cellule en dehors du réservoir de produit (17),
dans lequel le réservoir de lavage (16) comprend un troisième plongeur (28) sur sa section supérieure pour expulser les microcapsules de support de cellule, le plongeur (28) ayant une forme de fond complémentaire à une forme de fond du réservoir de lavage (16).

2. Appareil de lavage selon la revendication 1,
dans lequel le réservoir de lavage (17) présente une première sortie (21),
le réservoir de lavage (17) étant raccordé à sa première sortie (21) vers une entrée (11) d'un réservoir de déchets (10).

3. Appareil de lavage selon la revendication 2,
dans lequel le réservoir de déchets (10) est raccordé au réservoir de lavage (16) par un filtre (22), le filtre (22) ayant de préférence une taille de pore inférieure à 50 µm, de façon plus préférée de 20 à 40 µm, de façon la plus préférée d'environ 30 µm.

4. Appareil de lavage selon l'une des revendications 1 à 3,
dans lequel le réservoir de lavage (16) présente une seconde sortie (23) pour le raccordement sur une cartouche (24) pour recevoir les microcapsules de support de cellule.

5. Appareil de lavage selon la revendication 4,
dans lequel le troisième plongeur (28) facilite le transport des microcapsules de support de cellule depuis le réservoir de lavage (16) vers la cartouche (24).

6. Appareil de lavage selon l'une des revendications 1 à 5,
dans lequel il est prévu un tube de dérivation (27) raccordant le réservoir de stockage (6) au réservoir de lavage (16).

7. Appareil de lavage selon l'une des revendications 2 à 6,
dans lequel il est prévu un tube de trop-plein (26) raccordant une cartouche (24) au réservoir de déchets (10).

8. Appareil de lavage selon l'une des revendications 2 à 7,
dans lequel le réservoir de déchets (10) comprend une ouverture avec un filtre (14) pour la purge, le filtre (14) ayant de préférence une taille de pore de 0,1 à 1,0 µm, de façon plus préférée d'environ 0,15 à 0,3 µm, de façon la plus préférée d'environ 0,2 µm.
